# EUROPEAN PATENT SPECIFICATION

(11) **EP 1 360 933 B1**
(45) Date of publication and mention of the grant of the patent: **12.07.2006**
(21) Application number: 03252882.0
(22) Date of filing: 08.05.2003
(51) Int. Cl.: A61B 5/15

(54) **Physiological sample collection devices and methods of using the same**
Verfahren und Vorrichtung zur Sammlung von physiologischen Proben
Appareils et méthodes d'emploi de collecte d'échantillons physiologiques

(30) Priority: 09.05.2002 US 143442
(43) Date of publication of application: 12.11.2003
(73) Proprietor: LifeScan, Inc., Milpitas, CA 95035 (US)
(72) Inventor: Yuzhakov, Vadim, San Jose, California 95127 (US); McAllister, Devin, Shrewsbury Massachusetts 01545 (US); Olson, Lorin, Scotts Valley, California 95066 (US); Leong, Koon-wah, Sunnyvale, California 94086 (US); Teodorczyk, Maria, San Jose, California 95135 (US)
(74) Representative: Mercer, Christopher Paul

(56) References cited:
- WO-A-01/72220
- US-A- 4 637 403
- US-A- 5 700 695
- US-A- 5 938 679
- US-A- 6 036 924

## Description

### FIELD OF THE INVENTION

The invention relates to the collection of physiological samples and the determination of analyte concentrations therein.

### BACKGROUND OF THE INVENTION

Analyte concentration determination in physiological samples is of ever increasing importance to today's society. Such assays find use in a variety of application settings, including clinical laboratory testing, home testing, etc., where the results of such testing play a prominent role in the diagnosis and management of a variety of disease conditions. Analytes of interest include glucose for diabetes management, cholesterol for monitoring cardiovascular conditions, and the like. In response to this growing importance of analyte concentration determination, a variety of analyte concentration determination protocols and devices for both clinical and home testing have been developed.

In determining the concentration of an analyte in a physiological sample, a physiological sample must first be obtained. Obtaining the sample often involves cumbersome and complicated devices which may not be easy to use or may be costly to manufacture. Furthermore, the procedure for obtaining the sample may be painful. For example, pain is often associated with the size of the needle used to obtain the physiological sample and the depth to which the needle is inserted. Depending on the analyte and the type of test employed, a relatively large, single needle or the like is often used to extract the requisite amount of sample.

The analyte concentration determination process may also involve a multitude of steps. First, a sample is accessed by use of a skin-piercing mechanism, *e.g*., a needle or lancet, which accessing may also involve the use of a sample collection mechanism, *e.g*., a capillary tube. Next, the sample must then be transferred to a testing device, *e.g*., a test strip or the like, and then oftentimes the test strip is then transferred to a measuring device such as a meter. Thus, the steps of accessing the sample, collecting the sample, transferring the sample to a biosensor, and measuring the analyte concentration in the sample are often performed as separate, consecutive steps with various device and instrumentation.

Because of these disadvantages, it is not uncommon for patients who require frequent monitoring of an analyte to simply become non-compliant in monitoring themselves. With diabetics, for example, the failure to measure their glucose level on a prescribed basis results in a lack of information necessary to properly control the level of glucose. Uncontrolled glucose levels can be very dangerous and even life threatening.

Attempts have been made to combine a lancing-type device with various other components involved in the analyte concentration determination procedure in order to simplify the assay process. For example, U.S. Patent No. 6,099,484 discloses a sampling device which includes a single needle associated with a spring mechanism, a capillary tube associated with a pusher, and a test strip. An analyzer may also be mounted in the device for analyzing the sample. Accordingly, the single needle is displaced toward the skin surface by un-cocking a spring and then retracting it by another spring. A pusher is then displaced to push the capillary tube in communication with a sample and the pusher is then released and the fluid is transferred to a test strip.

U.S. Patent No. 5,820,570 discloses an apparatus which includes a base having a hollow needle and a cover having a membrane, whereby the base and cover are connected together at a hinge point. When in a closed position, the needle is in communication with the membrane and fluid can be drawn up through the needle and placed on the membrane of the cover.

There are certain drawbacks associated with each of the above devices and techniques. For example, the devices disclosed in the aforementioned patents are complex, thus decreasing ease-of-use and increasing manufacturing costs. Furthermore, as described, a single needle design may be associated with increased pain because the single needle must be large enough to extract the requisite sample size. Still further, in regards to the '484 patent, the steps of activating and retracting a needle and then activating and retracting a capillary tube adds still more user interaction and decreases ease-of-use.

As such, there is continued interest in the development of new devices and methods for use in the determination of analyte concentrations in a physiological sample. Of particular interest would be the development of integrated devices, and methods of use thereof, that are efficient, involve minimal pain, are simple to use and which may be used with various analyte concentration determination systems. However, in producing such devices the present invention places particular emphasis on issues associated with manufacturing and distribution, thereby offering more cost effective and flexible options, both to consumers and manufactures.

### SUMMARY OF THE INVENTION

Devices, systems and methods are provided for piercing the skin, accessing and collecting physiological sample therein, and measuring a characteristic of the physiological sample. The subject devices include at least one microneedle or skin-piercing element affixable to a test strip. The subject test strips include a biosensor, wherein the at least one skin-piercing element is separately attached to the biosensor. Document WO 01/72220 discloses such a device.

The Preferred skin-piercing elements of the invention have a space-defining configuration in which, upon insertion into the skin, creates a space or volume within the pierced tissue. This space serves as a reservoir or pooling area within which bodily fluid is caused to pool while the skin-piercing element is *in situ*. A capillary channel or fluid pathway extending from the pooling space to within the test strip transfers pooled fluid present within the pooling space to the biosensor. In certain embodiments, the space-defining configuration is a recess within a surface of the skin-piercing element. Such a recess may have a concave configuration. In other embodiments, the space-defining configuration is an opening which extends transverse to a dimension of the skin-piercing element and occupies a substantial portion of a width or diameter dimension as well as a substantial portion of a length dimension of the microneedle.

Generally, test strips used in connection with the needle or lance members of the present inventions may include electrochemical or photometric/colorimetric sensors. Other types of test strips may be used as well.

Needles or lance members according to the present invention may be affixed to test strips members in a number of ways. They may be affixed directly, *e.g*., using adhesive, chemical or ultrasonic welding. Alternately, mechanical attachment via clips hasps or the like may be employed.

Numerous advantages are presented in so-producing completed test strips/lances member combinations.

The subject systems include one or more subject test strip devices and a meter for receiving a subject test strip and for determining a characteristic of the sampled fluid, e.g., the concentration of at least one analyte in the sample, collected by within the test strip's biosensor. Moreover, such a meter may also provide means for activating and manipulating the test strip wherein the skin-piercing structure is caused to pierce the skin. Additionally, the meter may provide means for storing one or more subject test strips, or a cartridge containing a plurality of such test strips.

Also provided are methods for using the subject devices, as well as kits that include the subject devices and/or systems for use in practicing the subject methods. The subject devices, systems and methods are particularly suited for collecting physiological sample and determining analyte concentrations therein and, more particularly, glucose concentrations in blood, blood fractions or interstitial fluid. The present invention further includes methods for fabricating the subject test strip devices, in which a microneedle or skin-piercing element is affixed to a complete/discrete test strip unit. The subject fabrication methods may be used to fabricate individual test strip devices or a plurality of such test strip devices on a web, film or sheet of suitable material.

These and other objects, advantages, and features of the invention will become apparent to those persons skilled in the art upon reading the details of the methods and systems of the present invention which are more fully described below.

### BRIEF DESCRIPTION OF THE DRAWINGS

Each of the following figures diagrammatically illustrate aspects of the present invention. Variation of the invention from that shown in the figures is contemplated.

Figure 1 is a perspective view of a representative meter as may be used in connection with variations of the present invention.

Figures 2A and 3A are perspective views of the invention as used in colorimetric test devices; figures 2B and 3B are perspective views of lance members to be attached to test strips by adhesive and mechanical fasteners.

Figures 4A and 4B are perspective hidden-line views of the invention as used in electrochemical test devices, wherein plastic and metal lance member are shown.

Figures 5A is an exploded perspective view of an alternate lance configuration employing dispersion channels; figure 5B is a perspective view of the components in FIG 4A shown assembled from below.

Figure 6 is a perspective view of an alternate lance member resembling that in FIGs 5A and 5B, but provided in a low-profile format.

Figure 7 is a perspective view of yet another lance member, this one employing an inset dispersion zone.

### DETAILED DESCRIPTION OF THE INVENTION

In describing the invention in greater detail than provided in the Summery above, colorimetric and electrochemical test strips sensors are first described, followed by discussion of features and the use of exemplary combination test strip meter and lancing device of the present invention. Then, the manner in which colorimetric and electrochemical test strip may be provided in connection with examples of the present invention is set forth. This description is followed by disclosure of various alternate lance/needle member configurations. Then, methods of manufacture and kits advantageously incorporating components of the present invention are described.

Before the present invention is described in such detail, however, it is to be understood that this invention is not limited to particular variations set forth and may, of course, vary. Various changes may be made to the invention described and equivalents may be substituted without departing from the true spirit and scope of the invention. In addition, many modifications may be made to adapt a particular situation, material, composition of matter, process, process act(s) or step(s), to the objective(s), spirit or scope of the present invention. All such modifications are intended to be within the scope of the claims made herein. For example, description of the use of electrochemical and photometric sensor type test strips is not intended to be limiting; those skilled in the art will appreciate that the subject devices, systems and methods are useful in the measurement of other physical and chemical characteristics of biological substances, e.g., blood coagulation time, blood cholesterol level, *etc.*

Methods recited herein may be carried out in any order of the recited events which is logically possible, as well as the recited order of events. Furthermore, where a range of values is provided, it is understood that every intervening value, between the upper and lower limit of that range and any other stated or intervening value in that stated range is encompassed within the invention. Also, it is contemplated that any optional feature of the inventive variations described may be set forth and claimed independently, or in combination with any one or more of the features described herein.

All existing subject matter mentioned herein (*e.g.*, publications, patents, patent applications and hardware) is incorporated by reference herein in its entirety except insofar as the subject matter may conflict with that of the present invention (in which case what is present herein shall prevail). The referenced items are provided solely for their disclosure prior to the filing date of the present application. Nothing herein is to be construed as an admission that the present invention is not entitled to antedate such material by virtue of prior invention.

Reference to a singular item, includes the possibility that there are plural of the same items present. More specifically, as used herein and in the appended claims, the singular forms "a," "and," "said" and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Finally, it is noted that unless defined otherwise herein, all technical and scientific terms used herein have the same meaning as commonly understood by one of ordinary skill in the art to which this invention belongs.

### Colorimetric/Photometric Sensor Variations

In testers including colorimetric or photometric (herein used interchangeably) biosensor, the same is provided by at least a matrix and/or a membrane for receiving a sample and a reagent composition (set within the matrix or membrane) set upon a support structure. Where a membrane as well as a matrix is provided, the membrane will generally be placed opposite of the support structure upon the matrix. A membrane advantageously includes apertures or pores for sample access.

In some embodiments, the sensor comprises a membrane containing a reagent composition impregnated therein while a matrix may or may not contain reagent composition. Often the matrix preferably provides a deposition area for the various members of the signal producing system, described *infra*, as well as for the light absorbing or chromogenic product produced by the signal producing system, *i.e*., the indicator, as well as provides a location for the detection of the light-absorbing product produced by the indicator of the signal producing system.

A membrane provided may comprise a membrane that exhibits aqueous fluid flow properties and is sufficiently porous (*i.e*., provides sufficient void space) for chemical reactions of a signal producing system to take place. Ideally, the membrane pore structure would not support red blood cell flow to the surface of the membrane being interrogated (*i.e*., the color intensity of which is a subject of the measurement correlated to analyte concentration). Any matrix provided may or may not have pores and/or a porosity gradient, *e.g.* with larger pores near or at the sample application region and smaller pores at the detection region.

Materials from which a membrane may be fabricated vary, include polymers, *e.g.* polysulfone, polyamides, cellulose or absorbent paper, and the like, where the material may or may not be functionalized to provide for covalent or non-covalent attachment of the various members of the signal producing system. In a tester made a thin membrane material, the tester may require less than 1/2 µl of sample to wet a sufficiently large area of the membrane to obtain a good optical measurement.

Regarding suitable matrices, a number of different types have been developed for use in various analyte detection assays, which matrices may differ in terms of materials, dimensions and the like, where representative matrices include, but are not limited to, those described in U.S. Patent Nos.: 4,734,360; 4,900,666; 4,935,346; 5,059,394; 5,304,468; 5,306,623; 5,418,142; 5,426,032; 5,515,170; 5,526,120; 5,563,042; 5,620,863; 5,753,429; 5,573,452; 5,780,304; 5,789,255; 5,843,691; 5,846,486; 5,968,836 and 5,972,294; the disclosures of which are herein incorporated by reference.

However configured, one or more members of a signal producing system of the biosensor produce a detectable product in response to the presence of analyte, which detectable product can be used to derive the amount of analyte present in the assayed sample. In the subject test strips, the one or more members of the signal producing system are preferably associated with (*e.g*., covalently or non-covalently attached to) at least a portion of (*i.e*., the detection region) the matrix or membrane, and in many embodiments to substantially all of the same.

The signal producing system may comprise an analyte oxidation signal producing system. By analyte oxidation signal producing system, it is meant that in generating the detectable signal from which the analyte concentration in the sample is derived, the analyte is oxidized by a suitable enzyme to produce an oxidized form of the analyte and a corresponding or proportional amount of hydrogen peroxide. The hydrogen peroxide is then employed, in turn, to generate the detectable product from one or more indicator compounds, where the amount of detectable product generated by the signal measuring system, *i.e.* the signal, is then related to the amount of analyte in the initial sample. As such, the analyte oxidation signal producing systems present in the subject test strips are also correctly characterized as hydrogen peroxide based signal producing systems.

Hydrogen peroxide based signal producing systems include an enzyme that oxidizes the analyte and produces a corresponding amount of hydrogen peroxide, where by corresponding amount is meant that the amount of hydrogen peroxide that is produced is proportional to the amount of analyte present in the sample. The specific nature of this first enzyme necessarily depends on the nature of the analyte being assayed but is generally an oxidase or dehydrogenase. As such, the first enzyme may be: glucose oxidase (where the analyte is glucose), or glucose dehydrogenase either using NAD or PQQ as cofactor; cholesterol oxidase (where the analyte is cholesterol); alcohol oxidase (where the analyte is alcohol); lactate oxidase (where the analyte is lactate) and the like. Other oxidizing enzymes for use with these and other analytes of interest are known to those skilled in the art and may also be employed. In those preferred embodiments where the reagent test strip is designed for the detection of glucose concentration, the first enzyme is glucose oxidase. The glucose oxidase may be obtained from any convenient source (*e.g.* a naturally occurring source such as Aspergillus niger or Penicillum, or recombinantly produced).

The second enzyme of such a signal producing system is an enzyme that catalyzes the conversion of one or more indicator compounds into a detectable product in the presence of hydrogen peroxide, where the amount of detectable product that is produced by this reaction is proportional to the amount of hydrogen peroxide that is present. This second enzyme is generally a peroxidase, where suitable peroxidases include: horseradish peroxidase (HRP), soy peroxidase, recombinantly produced peroxidase and synthetic analogs having peroxidative activity and the like. See, *e.g.*, Y. Ci, F. Wang; Analytica Chimica Acta, 233 (1990), 299-302.

Indicator compound or compounds provided are preferably ones that are either formed or decomposed by the hydrogen peroxide in the presence of the peroxidase to produce an indicator dye that absorbs light in a predetermined wavelength range. Preferably the indicator dye absorbs strongly at a wavelength different from that at which the sample or the testing reagent absorbs strongly. The oxidized form of the indicator may be a colored, faintly-colored, or colorless final product that evidences a change in color of the testing side of the membrane. That is to say, the testing reagent can indicate the presence of glucose in a sample by a colored area being bleached or, alternatively, by a colorless area developing color.

Indicator compounds that are useful in the present invention include both one- and two-component chromogenic substrates. One-component systems include aromatic amines, aromatic alcohols, azines, and benzidines, such as tetramethyl benzidine-HCl. Suitable two-component systems include those in which one component is MBTH, an MBTH derivative (see *e.g.*, those disclosed in EP-A-0 781 350, or 4-aminoantipyrine and the other component is an aromatic amine, aromatic alcohol, conjugated amine, conjugated alcohol or aromatic or aliphatic aldehyde. Exemplary two-component systems are 3-methyl-2-benzothiazolinone hydrazone hydrochloride (MBTH) combined with 3-dimethylaminobenzoic acid (DMAB); MBTH combined with 3,5-dichloro-2-hydroxybenzene-sulfonic acid (DCHBS); and 3-methyl-2-benzothiazolinone hydrazone N-sulfonyl benzenesulfonate monosodium (MBTHSB) combined with 8-anilino-1 naphthalene sulfonic acid ammonium (ANS). In certain embodiments, the dye couple MBTHSB-ANS is preferred.

In yet other embodiments of colorimetric sensors that may be used in the present invention, signal producing systems that form a fluorescent detectable product (or detectable non- fluorescent substance, *e.g*. in a fluorescent background) may be employed, such as those described in Kiyoshi Zaitsu, Yosuke Ohkura, New fluorogenic substrates for Horseradish Peroxidase: rapid and sensitive assay for hydrogen peroxide and the Peroxidase, Analytical Biochemistry (1980) 109, 109-113. Examples of such colorimetric reagent test strips suitable for use with the subject invention include those described in U.S. Patent Nos. 5,563,042; 5,753,452; 5,789,255, herein incorporated by reference.

### Electrochemical Sensor Variations

Instead of using a colorimetric sensor as described above, the present invention may employ an electrochemical sensor. Typically, an electrochemical sensor comprises at least a pair of opposing electrodes, although electrochemical test strips with planar electrodes may be used in the present invention.

Where opposing-electrode type strips are employed, at least the surfaces of electrodes facing each other are comprised of a conductive layer such as a metal, where metals of interest include palladium, gold, platinum, silver, iridium, stainless steel and the like as well as carbon (conductive carbon ink) and indium doped tin oxide.

One conductive layer is preferably formed by sputtering a thin layer of gold (Au), the other by sputtering a thin layer of palladium (Pd). Alternately, the electrodes may be formed by screen printing a selected conductive pattern, including conductive leads, with a carbon or metal ink on the backing surfaces. An additional insulating layer may be printed on top of this conductive layer which exposes a precisely defined pattern of electrodes. However formed, after deposition of conductive layers, the surface may be subsequently treated with a hydrophilic agent to facilitate transport of a fluid sample into the reaction zone there between. Depending on the voltage sequence applied to the cell, one electrode may serve as a counter/reference electrode and the other as the working electrode of the electrochemical cell. However, where a double pulse voltage waveform is employed, each electrode acts as a counter/reference and working electrode once during analyte concentration measurement.

Regardless of reaction zone or electrode configuration, a reagent coating is typically provided therein. Reagent systems of interest typically include an enzyme and a redox active component (mediator). The redox component of the reagent composition, when present, is made up of one or more redox agents. A variety of different redox agents (*i.e*., mediators) are known in the art and include: ferricyanide, phenazine ethosulphate, phenazine methosulfate, pheylenediamine, 1-methoxy-phenazine methosulfate, 2,6-dimethyl-1,4-benzoquinone, 2,5-dichloro-1,4-benzoquinone, ferrocene derivatives, osmium bipyridyl complexes, ruthenium complexes, and the like. In many embodiments, the redox active component of particular interest is ferricyanide, and the like. The enzyme of choice may vary depending on the analyte concentration which is to be measured. For example, suitable enzymes for the assay of glucose in whole blood include glucose oxidase or dehydrogenase (NAD or PQQ based). Suitable enzymes for the assay of cholesterol in whole blood include cholesterol oxidase and esterase.

Other reagents that may be present in the reaction area include buffering agents (e.g., citraconate, citrate, malic, maleic, phosphate, "Good" buffers and the like); divalent cations (e.g., calcium chloride, and magnesium chloride); surfactants (e.g., Triton, Macol, Tetronic, Silwet, Zonyl, Aerosol, Geropon, Chaps, and Pluronic); and stabilizing agents (e.g., albumin, sucrose, trehalose, mannitol and lactose).

Examples of electrochemical biosensors suitable for use with the subject invention include those described in EP-A-1 067 384; EP-A-1 252 514; EP-A-1 254 365; WO 02/48707 and WO 02/50609.

### Test Strip Systems and Use

As mentioned above, the subject devices may be used in the context of a subject system, which generally includes a system capable of obtaining a physiological sample and determining a property of the sample, where determining the property of interest may be accomplished automatically by an automated device, *e.g*., a meter. The subject system is more particularly described herein in the context of analyte concentration determination. However, kits or systems according to the present invention include at least one subject test strip device **2**, oftentimes a plurality of test strip devices, where the at least one test strip device comprises at least on skin-piercing element **4**. The kits may also include a reusable or disposable meter **6** that may be used with disposable tests strip devices. Further, test strip kits may include a control solution or standard (*e.g*., a glucose control solution that contains a standardized concentration of glucose). A kit may also include instructions for using test strips according to the invention in the determination of an analyte concentration in a physiological sample. These instructions may be present on one or more of container(s), packaging, a label insert or the like associated with the subject test strips.

When a plurality of test strip devices is provided, they may be collectively packaged within a cartridge, which may be reusable or disposable. Certain of such kits may include various types of test strip devices, (*e.g*., electrochemical and/or colorimetric test strip devices). These various test strip devices may contain the same or different reagents.

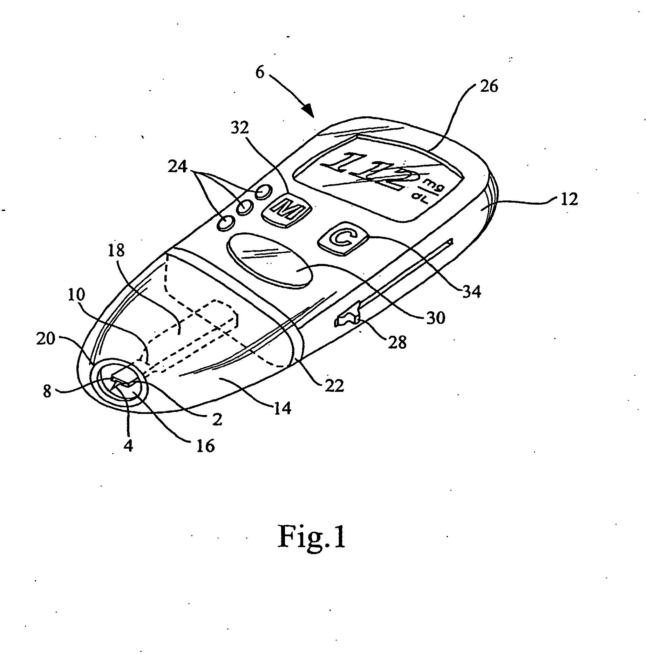

Regardless of the nature of the constituent components of any systems according to the present invention, the subject test strip devices, (whether electrochemical, colorimetric or otherwise), are preferably configured and adapted to be inserted into the meter. More specifically, as illustrated in FIG 1, test strip device **2** has a first end **8** and a second end **10,** wherein the skin-piercing or lancing blade or needle **4** is associated with first end **8** and at least the second end **10** is configured for insertion into meter **6.**

Meter **6** preferably has an ergonomically-designed housing **12** having dimensions which allow it to be comfortably held and manipulated with one hand. Housing **12** may be made of a metal, plastic or other suitable material, preferably one that is light weight but sufficiently durable. The distal portion **14** of the housing provides an aperture **16** through which test strip device **2** is advanced from a retracted position within meter **6** to an extended position wherein at least a portion of the test strip microneedle/lancet **4** extends a distance outside aperture **16**.

Distal portion **14** further defines a chamber in which test strip device **2** is received within a test strip receiving mechanism **18.** Test strip device **2** may be inserted into meter **6** by removing distal housing portion **14** from housing **12** and inserting test strip device **2** into test strip receiving mechanism **18**. Alternatively, test strip device **2** may be inserted into meter **6** and received into mechanism **18** via aperture **14.**

Preferably, distal housing portion **14** is transparent or semi-transparent to allow the user to visually confirm proper engagement between test strip device **2** and receiving area **18** prior to conducting the analyte concentration assay, as well as to visualize the test site and to visually confirm the filling of strip **2** with body fluid during the assay (especially if electronic sensing is not provided to discern the same) . When test strip device **2** is properly seated within receiving mechanism **18,** the biosensor with test strip device **2** operatively engages with the meter's testing components. In the case of electrochemical test strip embodiments, the electrodes of the biosensor operatively engage with the meter's electronics; with colorimetric test strip embodiments, the matrix or membrane area having a signal producing system is operatively aligned with the meter's optical components. The meter's electronics or optical componentry, upon sensing when the reaction zone or matrix area, respectively, within test strip device **2** is filled with the sampled fluid, supplies an input signal to the test strip biosensor and receives an output signal therefrom which is representative of the sample fluid characteristic being measured.

Circumferentially positioned about aperture **16** is a pressure ring **20,** the distal surface of which is applied to the skin and encircles the piercing site within the skin during a testing procedure. The compressive pressure exerted on the skin by pressure ring **20** facilitates the extraction of body fluids from the surrounding tissue and the transfer of such fluid into test strip device **2**.

Distal housing portion **14** is preferably itself in movable engagement with meter **6** wherein distal housing portion **14** is slightly translatable or depressible along a longitudinal axis of the meter. Between distal housing portion **14** and the a proximal portion of housing **12,** is a pressure sensor **22** which senses and gauges the amount of pressure exerted on distal housing portion **14** when compressing pressure ring **20** against the skin. Pressure sensor **22** is preferably an electrical type sensor which may be of the kind commonly known in the field of electronics. Pressure sensor indicators **24,** in electrical communication with pressure sensor **22,** are provided to indicate the level of pressure being applied to distal housing portion **14** so that the user may adjust the amount of pressure being applied, if necessary, in order to apply an optimal pressure.

In many embodiments, meter **6** has a display **26,** such as an LCD display, for displaying data, such as input parameters and test results. Additionally, meter **6** has various controls and buttons for inputting data to the meter's processing components and for controlling the piercing action of test strip device **2**. For example, lever **28** is used to retract test strip device **2** to a loaded position within meter **6** and thereby pre-load a spring mechanism (not shown) for later, on-demand extension or ejection of test strip device **2** from aperture **16** by depressing button **30.** When distal housing portion **04** is properly positioned on the skin, such ejection of test strip device **2** causes microneedle **4** to instantaneously pierce the skin for accessing the body fluid therein. Buttons **32** and **34,** when depressed, input signals to the meter's processing components indicating whether the measurement to be made is for testing/information purposes (and for recovering the test results from a memory means within the meter's electronics) or for calibration purposes, respectively.

Meter **6** may further be configured to receive and retain a replaceable cartridge containing a plurality of the subject test strip devices. After using a test strip device, the meter may either eject the used test strip from the meter or store them for disposal at a later time. Such a configuration eliminates the necessary handling of test strips, thereby minimizing the likelihood of damage to the strip and inadvertent injury to the patient. Furthermore, because manual handling of the test strips is eliminated, the test strips may be made much smaller thereby reducing the amount of materials required, providing a cost savings. The meter disclosed in copending European patent application No. , claiming priority from USSN 10/142 443 [Attorney ref: P033752EP], is of particular relevance in regard to these considerations.

Additionally, certain aspects of the functionality of meters suitable for use with the subject systems are disclosed in U.S. Patent No. 6,193,873, as well as in EP-A-1 252 514; EP-A-1 254 365; WO 02/48707; WO 02/50609; and EP-A-1 284 121. Of course, in those embodiments using a colorimetric assay system, a spectrophotometer or optical meter will be employed, where certain aspects of the functionality of such meters suitable for use are described in, for example, U.S. Patent Nos. 4,734,360, 4,900,666, 4,935,346, 5,059,394, 5,304,468, 5,306,623, 5,418,142, 5,426,032, 5,515,170, 5,526,120, 5,563,042, 5,620,863, 5,753,429, 5,773,452, 5,780,304, 5,789,255, 5,843,691, 5,846,486, 5,968,836 and 5,972,294.

In use, the subject invention provides methods for determining a characteristic of the sample, *e.g.*, the concentration of an analyte in a sample. The subject methods find use in the determination of a variety of different analyte concentrations, where representative analytes include glucose, cholesterol, lactate, alcohol, and the like. In many embodiments, the subject methods are employed to determine the glucose concentration in a physiological sample. Test devices **2** according to the present invention are particularly suited for use in determining the concentration of an analyte in blood or blood fractions, and more particularly in whole blood or interstitial fluid.

In practicing the subject methods, at least one subject test strip device as described above, is provided, and a subject microneedle **4** thereof is inserted into a target area of skin. Typically, the skin-piercing element is inserted into the skin of a finger or forearm for about 1 to 60 seconds, usually for about 1 to 15 seconds and more usually for about 1 to 5 seconds. Depending on the type of physiological sample to be obtained, the subject skin-piercing element **4** may be penetrated to various skin layers, including the dermis, epidermis and the stratum corneum, but in many embodiments will penetrate no farther than the subcutaneous layer of the skin.

While the subject test strips may be handled and inserted into the skin manually, the subject test strips are preferably used with a hand-held meter such as described above. As such, a single test strip device **2** is either initially inserted into test strip meter or the test strip may be provided by a pre-loaded cartridge (not shown). In the latter approach embodiment, the cartridge is preferably removably engageable with meter **6.** Used strips may be automatically disposed of, *e.g*., either ejected from the meter or deposited into a separate compartment within the cartridge, while an unused test strip is automatically removed from the cartridge and inserted into a receiving area of the meter.

Once test strip device **2** is properly received within mechanism **18,** it may then be spring loaded or cocked by means of lever **28**, thereby retracting the test strip device **2** and preparing it for firing. Meter **6** is then positioned substantially perpendicular to the targeted skin surface wherein distal housing portion **14,** and more specifically pressure ring **20,** is caused to contact the target skin area. Some compressive pressure may be manually applied to the target skin area, *i.e*., by pressing the distal end of meter **14** against the target skin area, to ensure that skin-piercing element **4** is properly inserted into the skin. By applying such pressure, a counter force causes distal housing portion **14** to press back upon pressure sensor **22**.

The relative amount (*i.e*., high, normal and low) of counter pressure is then measured and displayed by optional pressure sensor indicators **24.** Preferably, the amount of pressure applied should generally be in the "normal" range. Indicators **24** inform the user as to when too much or too little pressure is being applied. When the indicators show that the applied pressure is "normal", the user may then depress the spring-release button **30.** Due to the spring force released, receiving/carrying mechanism **18** and test strip device **2** are caused to thrust forward thereby causing skin-piercing element **4** to extend from aperture **16** and puncture the targeted skin area.

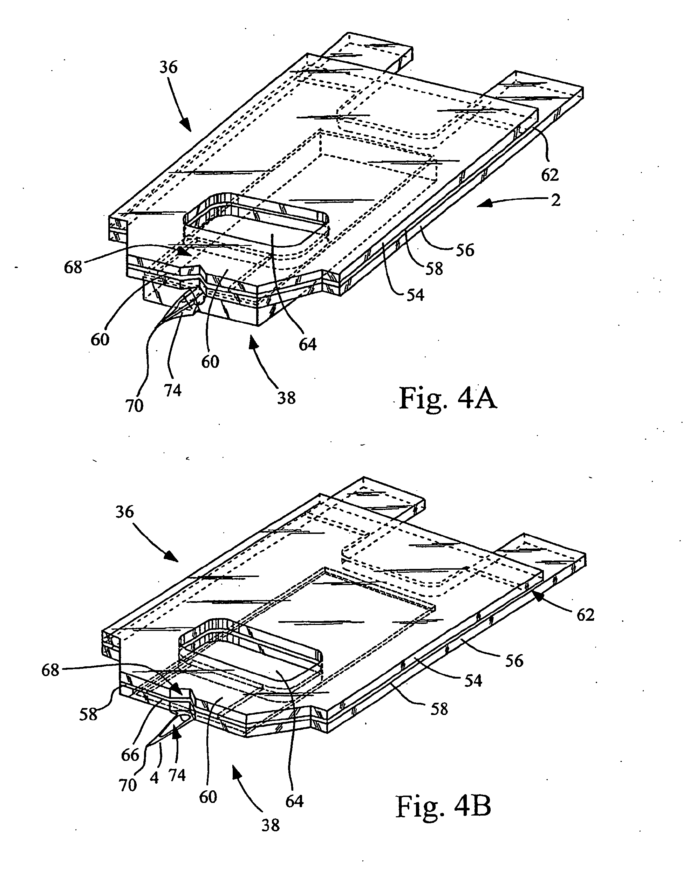

Whether by manual means or by use of meter **6,** the penetration of skin-piercing element **4** into the skin may create a fluid sample pooling area (defined by the recess or opening within skin-piercing element variations shown in FIGs 4A-7 and described further therewith). In which case, sample fluid enters the pooling area by the open-space configuration (*e.g*., recess or opening, within skin piercing element 4), and possibly also from the opposite side of the skin-piercing element. The pooled sample fluid is then transferred directly to the reaction zone of a test strip or thereto by a fluid pathway by at least a capillary force exerted on the pooled fluid. Where no enlarged pooling area is provided, a simple capillary channel may prove effective in certain situations as well, though such a set-up may not be most preferred.

In any case, the transfer of fluid from the wound site to the biosensor may be further facilitated by exerting physical positive pressure circumferentially around the penetration site by means of a pressure ring **20** or by applying a source of negative pressure through the fluid channel thereby vacuuming the body fluid exposed to the distal end of the channel. Fluid passing into the biosensor reaction zone may simply fill the area or alternately be distributed by subchannels or another similar distribution feature.

Once meter **6** senses that the reaction zone or matrix area is completely filled with the sample of body fluid, the meter electronics or optics are activated to perform analysis of the extracted sample. At this point, the meter may be removed by the patient from the penetration site or kept on the skin surface until the test results are shown on the display. Meter **6** may alternatively or additionally include means for automatically retracting the microneedle strip from the skin once the reaction cell is filled with the body fluid sample.

With an electrochemical-based analyte concentration determination assay, an electrochemical measurement is made using the counter/reference and working electrodes. The electrochemical measurement that is made may vary depending on the particular nature of the assay and the meter with which the electrochemical test strip is employed, (*e.g.*, depending on whether the assay is coulometric, amperometric or potentiometric). Generally, the electrochemical measurement will measure charge (coulometric), current (amperometric) or potential (potentiometric), usually over a given period of time following sample introduction into the reaction area. Methods for making the above described electrochemical measurement are further described in U.S. Patent Nos.: 4,224,125; 4,545,382; and 5,266,179; as well as in International Patent Publications WO 97/18465 and WO 99/49307.

Following detection of the electrochemical signal generated in the reaction zone, the amount of the analyte present in the sample is typically determined by relating the electrochemical signal generated from a series of previously obtained control or standard values. In many embodiments, the electrochemical signal measurement steps and analyte concentration derivation steps, are performed automatically by a device designed to work with the test strip to produce a value of analyte concentration in a sample applied to the test strip. A representative reading device for automatically practicing these steps, such that user need only apply sample to the reaction zone and then read the final analyte concentration result from the device, is further described in EP-A-1 067 384.

For a colorimetric or photometric analyte concentration determination assay, sample applied to a subject test strip, more specifically to a reaction area of a test strip, is allowed to react with members of a signal producing system present in the reaction zone to produce a detectable product that is representative of the analyte of interest in an amount proportional to the initial amount of analyte present in the sample. The amount of detectable product (*i.e*., signal produced by the signal producing system) is then determined and related to the amount of analyte in the initial sample. With such colorimetric assays, optical-type meters are used to perform the above mentioned detection and relation steps. The above described reaction, detection and relating steps, as well as instruments for performing the same, are further described in U.S. Patent Nos. 4,734,360; 4,900,666; 4,935,346; 5,059,394; 5,304,468; 5,306,623; 5,418,142; 5,426,032; 5,515,170; 5,526,120; 5,563,042; 5,620,863; 5,753,429; 5,773,452; 5,780,304; 5,789,255; 5,843,691; 5,846,486; 5,968,836 and 5,972,294; the disclosures of which are herein incorporated by reference. Examples of such colorimetric or photometric reagent test strips suitable for use with the subject invention include those described in U.S. Patent Nos.: 5,563,042; 5,753,452; 5,789,255, herein incorporated by reference.

### Test Strip Devices

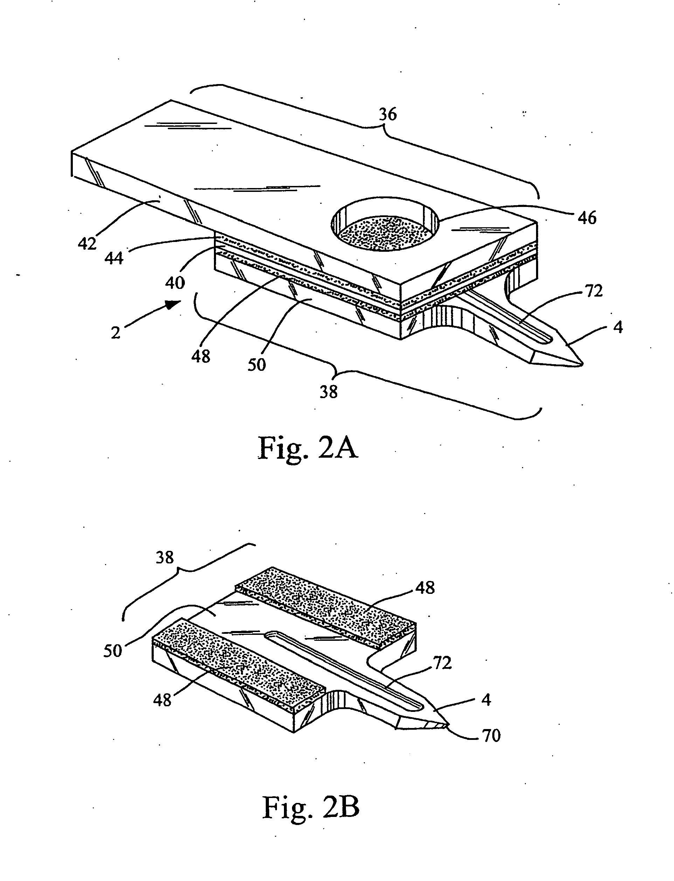

Turning now to FIGS. 2A and 2B, a first test element or tester **2** is shown. It comprises a test strip **36** and a needle/microneedle or lance/lancet portion **38** (herein used interchangeably). FIG 2B shows the lance element **38** shown separately, whereas a discrete test strip **36** and lance element **38** and are affixed, held or attached to each other in FIG 2A to form tester **2**

The test strip includes a biosensor **40** set upon a substrate **42.** Adhesive member(s) **44** may be provided to make the connection. The biosensor shown in FIG 2A is a colorimetric-type sensor provided in connection with a membrane and/or matrix. An aperture or transparent window **46** may be provided in substrate **42** to enable sensor reading.

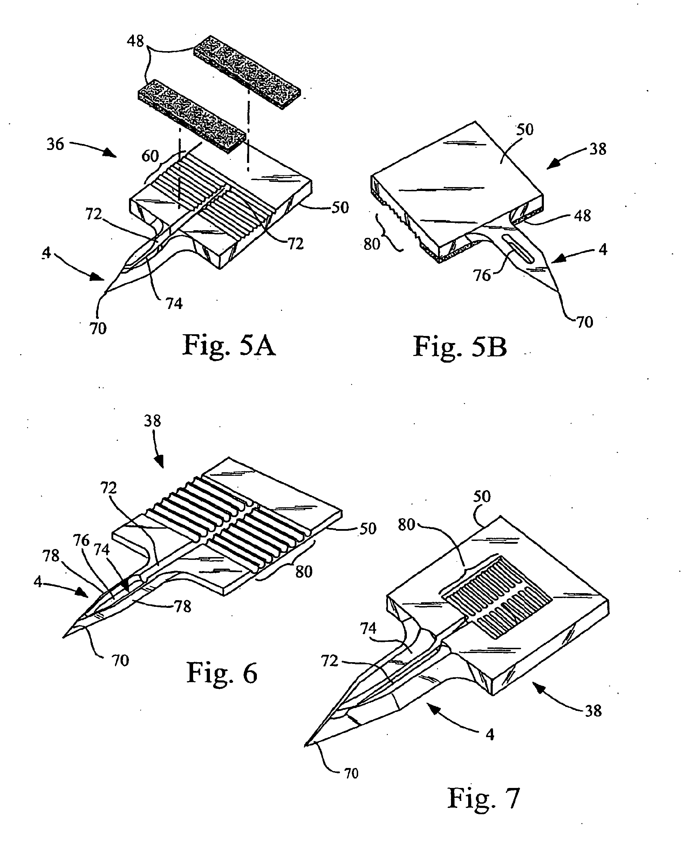

To attach the lance element in FIG 2B to the test strip in FIG 2A adhesive member(s) **48** are applied to a base **50** of the lance element to connect it to an opposing portion of the test strip. The orientation of such members may, of course, vary. Generally they will be set so as not to interfere with relevant structure. FIGs 5A and 5B provide an example of alternate adhesive portion placement used to attach the lance element to a test strip.

Regardless of relative orientation or configuration, as with optional adhesive portions **44**, adhesive portions **48** may comprise double-stick tape or directly-applied adhesive. Alternately, adhesive affixation of elements **36** and **38** may be foregone in favor of mechanically welding (for instance, using ultrasonics) or chemically welding the components together. Still further, supplemental attachment members may be provided to connect a test strip with a lance element according to the present invention.

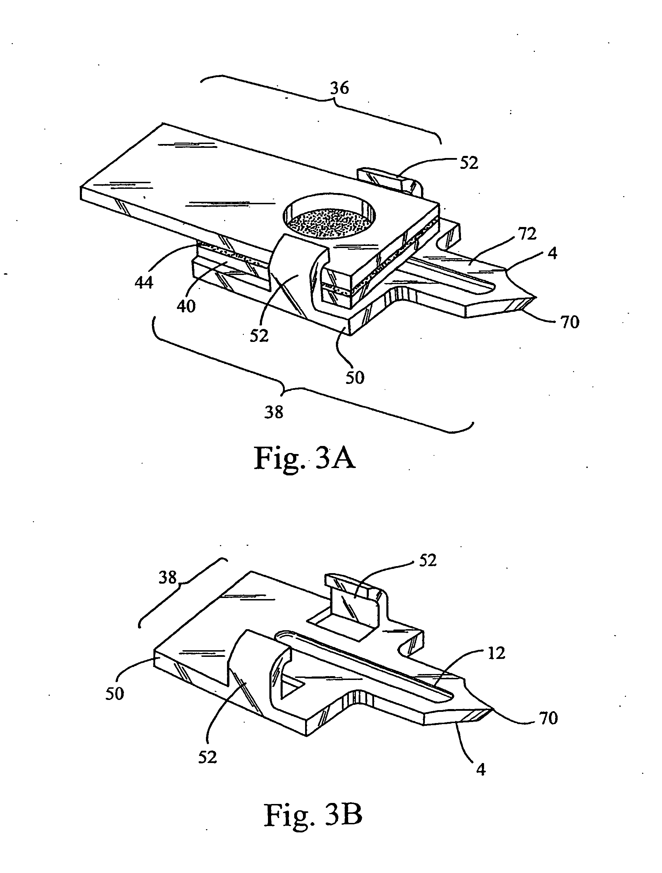

An example of such an approach is shown in FIGs 3A and 3B. Here, lance member **38** includes hooks or clasp members **52** provided on opposites sides of base **50.** The clips may be integrally formed in the lance element as shown, or comprise independent or discrete members themselves.

The variations of the invention in FIGs 4A and 4B are shown using adhered-on lance members **38** on their respective undersides. The base of each lance member may be affixed to the test strip body **36** by an adhesive layer or layers **44.** Of course clip-on lance members may alternately be used as may be other methods of connection.

As shown, the lance member in FIG 4A is of a different thickness than that in FIG 4B. This is because the former is sized to be made from plastic, while it is contemplated that the latter be produced from a metal. Indeed, any of the various lance member variations shown may alternately be made of either metal, plastic, composite material, ceramic or another material and be configured accordingly. Likewise, as may already be apparent, any of the attachment approaches described may be use in or with any of the lance member variations. Still further, each of the optional features regarding needle **4** structure and fluid conveyance as described further below may be used in each of the variations with either type of test strip **36** disclosed and still others.

However, details of the test strip embodiment in FIGs 4A and 4B is first described. Specifically, this test strip **36** comprises a first electrode **54** and a second electrode **56,** preferably constructed as described above in connection with electrochemical sensor production. The thickness of the any substrate material provided typically ranges from about 25 to 500 µm and usually from about 50 to 400 µm, while the thickness of the metal layer typically ranges from about 10 to 100 nm and usually from about 10 to 50 nm.

An adhesive member **58** may serve as a spacer between the electrodes, defining a reaction zone or area **60** for which the electrodes generally face each other and are separated by only a short distance, such that the spacing between the electrodes is extremely narrow. The thickness of spacer layer **58** may range from 10 to 750 µm and is often less than or equal to 500 µm, and usually ranges from about 25 to 175 µm. Any spacer layer preferably has double-sided adhesive to capture the adjacent electrodes. In any case spacer layer **58** may be fabricated from any convenient material, where representative suitable materials include polyethylene terephthalate, glycol modified polyethylene terephthalate (PETG), polyimide, polycarbonate, and the like.

As depicted, the working and reference electrodes are generally configured in the form of strips. Typically, the length of the electrodes rages from about 0.75 to 2 in (1.9 to 5.1 cm), usually from about 0.79 to 1.1 in (2.0 to 2.8 cm). The width of the electrodes ranges from about 0.15 to 0.30 in (0.38 to 0.76 cm), usually from about 0.20 to 0.27 in (0.51 to 0.67 cm). In certain embodiments, the length of one of the electrodes is shorter than the other, wherein in certain embodiments it is about 0.135 in (3.5 mm) shorter. Preferably, electrode and spacer width is matched where the elements overlap. The spacer incorporated in the strip may be set back about 0.3 in (7.6 mm) from the end of electrode **56,** leaving opening(s) **62** between the electrodes about 0.165 in (4.2 mm) deep. However, configured, such opening(s) provide space for receipt of a meter probe.

A vent opening **64** is provided across the reaction zone from the inlet port **66.** Providing a vent allows for capillary action between the electrodes to draw sample into the reaction zone without backpressure interference. Spacer layer **58** is preferably configured or cut-out so as to provide a reaction zone or area with a volume in the range from about 0.01 to 10 µL, usually from about 0.1 to 1.0 µL and more usually from about0.05 to 1.0 µL. The amount of physiological sample that is introduced into the reaction area of the test strip may vary, but generally ranges from about 0.1 to 10 µl, usually from about 0.3 to 0.6 µl.

Such introduction of sample is preferably accomplished at notched section **68**. It interfaces with features of needle **4** to pick up pooling or conveyed sample and direct it inwardly toward the test strip reaction zone, at least partially pinning the sample along the edges of the notch.

As such, the variations of the invention shown in FIGs 4A and 4B represent front-loaded test strips. Those in figures 2A and 2B are loaded with or accept sample along the face of the sensor (as present on the underside of the test strip). Still further modes of introduction are possible, however. Side loaded test strips may be employed (such as those described in EP-A-02258168.0 and EP-A-02258169.8) with minor modifications of the lance elements depicted. Such approaches are contemplated as part of the present invention.

### Lance Elements

Also contemplated as aspects of the present invention are various features regarding the lance elements **38** shown. In accordance with the text above, each lance element includes a lancet/needle or skin piercing element **4,** typically having a pointed tip **70.** In addition the body of lance **4** and base **50** may incorporate various features to collect and/or convey a biological sample to a given test strip sensor **40**.

Actually, any suitable shape of skin-piercing element **4** may be employed with the subject test strip devices, as long as the shape enables the skin to be pierced with minimal pain to the patient. For example, the skin-piercing element may have a substantially flat or planar configuration, or may be substantially cylindrical-like, wedge-like or triangular in shape such as a substantially flattened triangle-like configuration, blade-shaped, or have any other suitable shape. The cross-sectional shape of the skin-piercing element, or at least the portion of skin-piercing element that is penetrable into the skin, may be any suitable shape, including, but not limited to, substantially rectangular, oblong, square, oval, circular, diamond, triangular, star, *etc*. Additionally, the skin-piercing element may be tapered or may otherwise define a point or apex at its distal end. Such a configuration may take the form of an oblique angle at the tip or a pyramid or triangular shape or the like.

The dimensions of the skin-piercing element may vary depending on a variety of factors such as the type of physiological sample to be obtained, the desired penetration depth and the thickness of the skin layers of the particular patient being tested. Generally, the skin-piercing element is constructed to provide skin-piercing and fluid extraction functions and, thus, is designed to be sufficiently robust to withstand insertion into and withdrawal from the skin. Typically, to accomplish these goals, the ratio of the penetration length (defined by the distance between the base of the skin-piercing element and its distal tip) to diameter (where such diameter is measured at the base of the skin-piercing element) is from about 1 to 1, usually about 2 to 1, more usually about 5 to 1 or 10 to 1 and oftentimes 50 to 1.

The total length of the skin-piercing elements generally ranges from about 1 to 30,000 microns, usually from about 100 to 10,000 microns and more usually from about 1,000 to 3,000 microns. The penetration length of the skin-piercing elements generally ranges from about 1 to 5000 microns, usually about 100 to 3000 microns and more usually about 1000 to 2000 microns. The height or thickness of skin-piercing elements **38**, at least the thickness of the distal portion **4**, typically ranges from about 1 to 1000 microns, usually from about 10 to 500 microns and more usually from about 50 to 250 microns. The outer diameter at the base generally ranges from about 1 to 2000 microns, usually about 300 to 1000 microns and more usually from about 500 to 1000 microns. In many embodiments, the outer diameter of the distal tip generally does not exceed about 100 microns and is generally less than about 20 microns and more typically less than about 1 micron. However, it will be appreciated by one of skill in the art that the outer diameter of the skin-piercing element may vary along its length or may be substantially constant.

Regarding the fluid-conveying features noted as may be incorporated in lance element **38**, one variation incorporates only a channel **72,** preferably of capillary dimensions, for this purpose. Configured to work with the test strips in FIGs 2A and 3A, the channel preferably extends a sufficient length so that it is in fluid communication with the sensor matrix or membrane. The channel may be open on either one side (thereby taking the form of a trench) or both. The channel length is preferably limited to match-up with intended target in order to avoid inadvertent loss of sample fluid.

FIGs 4A and 4B show a somewhat different lance configuration. In each figure, a recessed polling area **74** is provided. No capillary is required to carry fluid from the pooling area since (as noted above) fluid is able to directly transfer from the lancet **4** to access port **66** in this variation of the invention. The purpose of the recessed or space-defining area in the variations shown in FIGs 4A and 4B (as well as in FIGs 5A-7) is to create a space or volume within the pierced tissue. This space serves as a reservoir within which bodily fluid is caused to pool *in situ* prior to being transferred to the biosensor portion of the subject test strip devices. As such, the availability of a greater volume of body fluid can be provided with a tip that is smaller and/or sharper than conventional microneedles, thereby reducing pain. The greater availability of body fluid also results in a faster collection rate of sampling.

Generally, the space-defining lancet configurations of the present invention create or define a space within the pierced tissue having a volume at least as great as the available fluid volume in the reaction zone of the biosensor. Such space or volume ranges from about 10 to 1,000 nL, and more usually from about 50 to 250 nL. Such volume occupies a substantial portion of the entire volume occupied by the structure of the skin-piercing element, and ranges from about 50% to 99% and more usually from about 50% to 75% of the entire volume occupied by the skin piercing element.

The lance member variations shown in FIGs 5A-7 incorporate a channel **72** and a recess **74.** The variations in FIGs 5 and 6 include an opening **76** adjacent the pooling region as well. The pooling area opening in the former variations is best pictured in FIG 5B. The purpose of such an opening (and for providing an open capillary in the lance member variations referenced above from FIGs 2A-3B) is to further expose the sample-gathering structure area to the outside environment, thereby increasing the volume and flow rate of body fluid into the area.

As illustrated, the recesses and/or openings may occupy a substantial portion of the width of their respective skin-piercing elements, as well as a substantial portion of a length dimension. Side walls **78** defining each of the structures will have a thickness sufficient to maintain the structure of the microneedle when subject to normal forces, but may be minimized in order to maximize negative space for collecting sample.

Another optional feature or set of features that may be employed, especially in connection with a fluid conveying channel **72** incorporated in a lance element is shown in each of FIGs 5A-7. The features being referred to are the secondary fluid transfer pathways **80**. These elements, set in fluid communication with channel **72** convey sample outwardly, dispersing the same across the sensor employed in an opposing, attached test strip.

Like channel **72,** pathways or channels **80** are preferably dimensioned so as to exert a capillary force on fluid within the pooling area defined by the open space portion of the microneedle, and draws or wicks physiological sample to within the reaction zone or matrix area of the biosensor. As such, the diameter or width of a single fluid channel or pathway does not exceed 1000 microns and will usually be about 100 to 200 microns in diameter. This diameter may be constant along its length or may vary. It may be preferred that sub-channels **80** have cross-sectional diameters in the range from about 1 to 200 microns and more usually from about 20 to 50 microns in that they are not required to convey the same volume of fluid as a primary channel **72.**

In the illustrated embodiments, branch channels **80** extend perpendicularly from channel **72**; however, they may extend angularly from their respective channels. Another variation concerning lance member configuration relative to channels **80** is to inset or surround the same within base as shown in FIG 7. Accomplished in this manner or another way, bounding the area to which channels **80** can convey fluid can be employed to ensure that sample is directed fully and only to a reaction or sensor area of the test strip **36** employed with lance element **38**.

In certain embodiments of the invention, the fluid pathway may further include one or more agents to facilitate sample collection. For example, one or more hydrophilic agents may be present in the fluid pathway, where such agents include, but are not limited to types of surface modifiers or surfactants such as mercaptoethane sulfonic acid (MESA), Triton, Macol, Tetronic, Silwet, Zonyl, Aerosol, Geropon, Chaps, and Pluronic.

### Test Strip Device Fabrication

Many of the techniques described in European Patent Application No. , claiming priority from USSN 10/143399 filed 9th May 2002, [Attorney ref: P033762EP] are applicable to fabricating test strip devices as described herein - especially those details regarding needle/lance production. Details as to electrochemical test strip production may also be appreciated in view of Application Atty Docket Nos. LIFE-031 entitled "SOLUTION DRYING SYSTEM" and LIFE-039 entitled "SOLUTION STRIPING SYSTEM".

A primary distinction, however, between the approach taught in the former application and that taught herein, is that in the present invention complete test strips may be provided, to which lance elements are attached as auxiliary structure. FIGs 2A and 3A provide examples of such an approach. Alternately, test strips adapted for use with the lance elements of the invention may be provided, to which lance elements are affixed. FIGs 4A and 4B provide examples of such an approach.

In either case, it is possible to separately produce or procure lance and test strip elements that are later brought together. The initially independent nature of the products/devices permits relatively optimized manufacture. In contrast, in the integral test strip devices described in the above-referenced application, certain considerations of material selection and manufacturing processes applicability that do not necessarily affect manufacture of the present invention.

One example of the flexibility offered by producing test strip devices according to the present invention by affixing a lance element to an otherwise complete test strip is that a user may feasibly take such action. This may be especially true for the clip-type embodiments disclosed (or variations of the embodiments shown in which clip-type structure maybe employed.) By virtue of such flexibility, there is market opportunity for selling lance members for use with any of a variety of commercially available test strips to be used with a meter according to the present invention. Of course, flexibility exists in designing the lance elements so they will interface (by clips, adhesive or other means) with a wide variety of test strips - both, present and future.

Though the invention has been described in reference to certain examples, optionally incorporating various features, the invention is not to be limited to the set-ups described. The invention is not limited to the uses noted or by way of the exemplary description provided herein. It is to be understood that the breadth of the present invention is to be limited only by the literal or equitable scope of the following claims.

## Claims

1. A lance element for attachment to a test strip to access body fluid and convey it to a test strip sensor, said lance comprising:
a substantially planar base;
a skin-piercing element comprising an opening for creating a pooling area within the skin of a subject upon insertion of said skin-piercing element into the skin, said opening occupying a substantial portion of a width, diameter or length dimension of said piercing element; and
a fluid pathway in communication with said opening for transporting body fluid present at said opening to the test strip.

2. The lance element of claim 1, wherein said fluid pathway is dimensioned to apply a capillary force on fluid present within said pooling area.

3. The lance element of claim 1, or claim 2 further comprising a recess within a surface of said base, wherein said recess is in fluid communication with said opening.

4. A test strip combination comprising:
a complete test strip comprising biosensor and a support member;
a separate lance element according to claim 1 attached to said test strip, said lance element being adapted to convey a fluid sample obtained by said piercing element to said biosensor.

5. The test strip combination of claim 4, wherein said test strip has an electrochemical configuration.

6. The test strip combination of claim 4, wherein said test strip has a photometric or colorimetric configuration.

7. The test strip combination of any one of claims 4 to 6, wherein said lance element is a lance element according to any one of claims 1 to 3.

8. A system for determining the concentration of at least one analyte in a physiological sample, said system comprising:
at least one test strip combination according to any one of claims 4 to 7, and
a meter for automatically determining the concentration of analyte in the physiological sample, wherein said meter is configured for receiving said test strip device.

9. A method of producing a tester, the method comprising:
providing a lance element as described in any one of claims 1 to 3, providing a test strip having a substrate and biosensor; and
attaching said lance element base to said test strip.

## Patentansprüche

1. Lanzettenelement zum Verbinden mit einem Teststreifen für den Zugriff auf Körperfluid und für dessen Leiten zu einem Teststreifensensor, wobei die Lanzette umfaßt:
- eine im wesentlichen ebene Basis,
- ein die Haut durchstechendes Element, welches eine Öffnung zum Erzeugen eines Sammelbereichs in der Haut eines Subjekts auf das Einftlhren des die Haut durclastechenden Elements in die Haut hin aufweist, wobei diese Öffnung einen wesentlichen Teil einer Breiten-, Durchmesser- oder Längenabmessung des durchstechenden Elements einnirnmt und
- einen Fluidweg in Verbindung mit der Öffnung zum Transportieren von Körpezfluid, das an dieser Öffnung vorhanden ist, zu dem Teststreifen.

2. Lanzettenelement nach Anspruch 1, bei welchem der Fluidweg so dimensioniert ist, daß eine Kapillarkraft auf Fluid wirkt, welches in dem Sammelbereich vorhanden ist.

3. Lanzettenelement nach Anspruch 1 oder Anspruch 2, welches weiterhin eine Vertiefung in einer Oberfläche der Basis aufweist, wobei die Vertiefung in einer Fluidverbindung mit der Öffnung steht.

4. Teststreifenkombination, welche umfaßt:
- einen kompletten Teststreifen, der einen Biosensor und ein Trageelement umfaßt,
- ein separates Lanzettenelement nach Anspruch 1, welches mit dem Teststreifen verbunden ist, wobei das Lanzettenelement dafür eingerichtet ist, eine Fluidprobe, die durch das durchstechende Element gewonnen wurde, zu dem Biosensor zu leiten.

5. Teststreifenkombination nach Anspruch 4, wobei der Teststreifen eine elekrochemische Konfiguration besitzt.

6. Teststreifenkombination nach Anspruch 4, wobei der Teststreifen eine photometrische oder eine kolorimetrische Konfiguration besitzt.

7. Teststreifenkombination nach einem der Ansprüche 4 bis 6, bei welchem das Lanzettenelement ein Lanzettenelement nach einem der Ansprüche 1 bis 3 ist.

8. System zum Bestimmen der Konzentration von mindestens einem Analyt in einer physiologischen Probe, wobei das System umfaßt:
- mindestens eine Teststreifenkombination nach einem der Ansprüche 4 bis 7 und
- ein Meßgerät zum automatischen Bestimmen der Konzentration von Analyt in der physiologischen Probe, wobei das Meßgerät zum Aufnehmen der Teststreifenvorrichtung konfiguriert ist.

9. Verfahren zum Herstellen eines Testgeräts, wobei das Verfahren umfaßt:
- Bereitstellen eines Lanzettenelements, wie es in einem der Ansprüche 1 bis 3 beschrieben ist,
- Bereitstellen eines Teststreifens mit einem Substrat und Biosensor und
- Verbinden der Basis des Lanzettenelements mit dem Teststreifen,

## Revendications

1. Elément de lancette destiné à être fixé à une bande de test pour accéder à un fluide corporel et le transporter vers un capteur de bande de test, ladite lancette comprenant :
une base sensiblement plane ;
un élément percutané comprenant une ouverture destinée à créer une zone de regroupement dans la peau d'un sujet lors de l'insertion dudit élément percutané dans la peau, ladite ouverture occupant une partie substantielle d'une dimension de largeur, de diamètre ou de longueur dudit élément percutané ; et
une trajectoire de fluide en communication avec ladite ouverture pour transporter un fluide corporel présent au niveau de ladite ouverture vers la bande de test.

2. Elément de lancette selon la revendication 1, dans lequel ladite trajectoire de fluide est dimensionnée pour appliquer une force capillaire sur le fluide présent dans la zone de regroupement.

3. Elément de lancette selon la revendication 1 ou la revendication 2, comprenant en outre un creux dans une surface de ladite base, dans lequel ledit creux est en communication de fluide avec ladite ouverture.

4. Combinaison de bande de test comprenant :
une bande de test complète comprenant un biocapteur et un élément de support ;
un élément de lancette distinct selon la revendication 1 fixé à ladite bande de test, ledit élément de lancette étant adapté pour transporter un échantillon de fluide obtenu par ledit élément percutané vers ledit biocapteur.

5. Combinaison de bande de test selon la revendication 4, dans laquelle ladite bande de test présente une configuration électrochimique.

6. Combinaison de bande de test selon la revendication 4, dans laquelle ladite bande de test présente une configuration photométrique ou colorimétrique.

7. Combinaison de bande de test selon l'une quelconque des revendications 4 à 6, dans laquelle ledit élément de lancette est un élément de lancette selon l'une quelconque des revendications 1 à 3.

8. Système destiné à déterminer la concentration d'au moins une substance à analyser dans un échantillon physiologique, ledit système comprenant :
au moins une combinaison de bande de test selon l'une quelconque des revendications 4 à 7, et
un dispositif de mesure destiné à déterminer automatiquement la concentration de substance à analyser dans l'échantillon physiologique, dans lequel ledit dispositif de mesure est configuré pour recevoir ledit dispositif de bande de test.

9. Procédé de production d'un testeur, le procédé comprenant les étapes consistant à :
fournir un élément de lancette selon l'une quelconque des revendications là 3,
fournir une bande de test comprenant un substrat et un biocapteur ; et
fixer la base dudit élément de lancette à ladite bande de test.
